# EUROPEAN PATENT SPECIFICATION

(11) **EP 2 412 362 B1**
(45) Date of publication and mention of the grant of the patent: **08.05.2019**
(21) Application number: 10755965.0
(22) Date of filing: 18.03.2010
(51) Int. Cl.: A61K 8/37, A61K 8/06, A61K 8/31, A61K 8/92, A61Q 19/00, A61Q 1/00

(54) **WATER-IN-OIL EMULSION COMPOSITION**
WASSER-IN-ÖL-EMULSIONSVERBINDUNG
COMPOSITION D'UNE ÉMULSION D'EAU DANS L'HUILE

(30) Priority: 27.03.2009 JP 2009079894
(43) Date of publication of application: 01.02.2012
(73) Proprietor: Shiseido Company, Ltd., Tokyo 104-0061 (JP)
(72) Inventor: IBE Ayako, Yokohama-shi Kanagawa 224-8558 (JP); WATANABE Kei, Yokohama-shi Kanagawa 224-8558 (JP); OMURA Takayuki, Yokohama-shi Kanagawa 224-8558 (JP)
(74) Representative: Lippert Stachow Patentanwälte Rechtsanwälte
(86) International application number: PCT/JP2010/054657
(87) International publication number: WO 2010/110167

(56) References cited:
- JP-A- 61 263 631
- JP-A- 2007 153 824
- JP-A- 2008 024 630
- JP-A- 2008 290 946
- JP-A- 2008 290 946
- US-A- 4 446 051
- US-A1- 2002 010 113
- US-B1- 6 730 642
- Anonymous: "GLYCEROL MONOISOSTEARATE | 66085-00-5", , 23 August 2016 (2016-08-23), XP055297120, Retrieved from the Internet: URL:http://www.chemicalbook.com/ChemicalPr oductProperty_EN_CB3330724.htm [retrieved on 2016-08-23]

## Description

### FIELD OF THE INVENTION

The present invention relates to a water-in-oil emulsion composition, and in particular, relates to the realization of a high moisturizing effect and the improvement of an elastic feeling and softness of the skin after application.

### BACKGROUND OF THE INVENTION

An emulsion is broadly-divided into an oil-in-water (O/W) type and a water-in-oil (W/O) type. In addition to these types, there are multi-type emulsions such as oil-in-water-in-oil (O/W/O) type and water-in-oil-in-water (W/O/W) type. Conventionally, these emulsions have been utilized in a skin-care cream, a milky lotion, and a hair-care cream, etc in the cosmetic field and a transdermal cream, etc in the pharmaceutical field.

Among them, a water-in-oil emulsion composition, in which an oil phase constitutes the outer phase and a water phase constitutes the inner phase, is a suitable form as an external skin preparation because oil-soluble active ingredients such as an emollient oil, an oil-soluble drug, and a UV absorber can be efficiently spread on the skin. In this regard, a water-in-oil emulsion is superior to an oil-in-water emulsion.

The internal-aqueous-phase proportion, which is obtained by dividing the amount of the internal-aqueous-phase component by the sum of the total aqueous phase component and the total oil phase component, has a large effect on the emulsion properties and on the feeling in use of the cosmetics containing the emulsion. Specifically, if the internal-aqueous-phase proportion is high in the water-in-oil emulsion composition used in a cream, a light-refreshing good feeling in use can be provided. If the internal-aqueous-phase proportion is low, a moist oily feeling is generated.

Although it depends on the selection of an oil component and an emulsifying agent, when the internal-aqueous-phase proportion of a normal water-in-oil emulsion composition is increased, it becomes difficult to maintain the stability in the vicinity of 60%. This is because the enlargement of emulsion particles takes place owing to the migration of water molecules, which constitute the emulsion particles of the internal aqueous phase, and the subsequent absorption into other emulsion particles (Ostwald ripening), and also because the coalescence of emulsion particles takes place owing to a significant increase in the collision frequency of emulsion particles due to a high internal-aqueous-phase proportion. Thus, it has been difficult to stabilize the emulsion at the internal-aqueous-phase proportion of more than 68%, which is approximately the closest packing rate of hard spheres (74%). In addition, the emulsion system is a thermodynamically non-equilibrium system. Therefore, in order to stabilize the emulsion for industrial application, there have been usage limitations in the amount of emulsifying agent, the internal-aqueous-phase proportion, the kinds and the amounts of aqueous components, the kinds and the amounts of the oil components, and the kinds and the amounts of other stabilizers.

That is, it has been difficult to provide a water-in-oil emulsion composition having a high moisturizing effect on the skin and having a high internal-aqueous-phase proportion that is suitable for the usability improvement while maintaining good stability.

On the other hand, a water-in-oil emulsion composition having a high internal-aqueous-phase proportion was developed by using mainly glycerol monooleate as the surfactant (for example, refer to patent literature 1). The above-described emulsion has freshness during application and a moisturizing effect after application. However, it has been difficult to provide a satisfactory elastic feeling and softness after application.

By using one or more surfactants selected from the group consisting of glycerol monooleate, glycerol monoisostearate, and polyoxyethylene glyceryl monoisostearate; and a solid oil component, a water-in-oil emulsion composition having freshness in use and a post-application moisturizing effect was developed (for example, refer to patent literature 2). By using glycerol monooleate and a surfactant having a branched chain in an alkyl group, a water-in-oil emulsion composition having a high moisturizing effect and high stability was also developed (for example, refer to patent literature 3). However, it was difficult even for these emulsions to provide a satisfactory elastic feeling and softness after application.

Furthermore, a water-in-liquid crystal emulsion composition with a high internal-aqueous-phase proportion, wherein discontinuous reverse-micellar cubic liquid crystals, which are packed with reverse micelles, are used as the outer phase and water is taken in as emulsion drops, was developed (for example, refer to non-patent literature 1). In this method, however, the kinds and the amounts of surfactants and oils are significantly limited; thus the method was non-practical and the storage stability was not satisfactory.

Patent literature 1: Japanese unexamined patent publication No. 2007-153824
Patent literature 2: Japanese unexamined patent publication No. 2008-24630
Patent literature 3: Japanese unexamined patent publication No. 2008-290946
Patent literature 4: US 4,446,051 A discloses a W/O emulsion comprising a mixed ester of diglycerol with isostearic and succinic acid (IMWITOR 780K), isoparaffine or branched chain light paraffine oil, respectively, and water.
Patent literature 5: EP 2165695 A1 discloses W/O emulsion compositions with a highly moisturizing effect on the skin comprising glyceryl monooleate, a surfactant, an aqueous component, an oil component and a perfume component.
Patent literature 6: US 6,730,642 B1 discloses a process for making a multiphase personal wash bar comprising solid components such as microcrystalline waxes and petrolatum.
Patent literature 7: US 2002/0010113 A1 discloses a composition useful for moisturizing skin comprising occlusive agents like hydrophobic oils and waxes.

Non-patent literature 1: Satsuki Kuribayashi, Oleoscience Vol.1. No.3, 247-254(2001)

### DISCLOSURE OF THE INVENTION

### PROBLEM TO BE SOLVED BY THE INVENTION

In the above-described conventional methods, the surfactant was elaborated to increase the internal-aqueous-phase proportion. According to the results, however, there was a usability problem in that a sticky sensation was present. In addition, the kinds of blendable oil components were limited in order to maintain stability while keeping a high internal-aqueous-phase proportion. Furthermore, when used in cosmetics, the post-application elastic feeling and softness of the skin were lacking; thus the quality was not necessarily satisfactory.

The present invention was made in view of the above-described problems of the conventional art. An object of the invention is to provide a water-in-oil emulsion composition that provides a post-application elastic feeling, softness, and a moisturizing effect and that is excellent in emulsion stability while it is a composition with a high internal-aqueous-phase proportion, which allows the makeup to stay on nicely because of its uniform applicability and provides a refreshing effect on the skin when used as a pre-makeup emulsion.

### MEANS TO SOLVE THE PROBLEM

The present inventors have diligently studied in view of the above-described problems of the conventional arts. As a result, the present inventors have found that a water-in-oil emulsion composition according to claim 1, comprising glycerol isostearate and/or glycerol oleate by mixing with the use of an isoparaffin having small number of carbon atoms, and fulfilling conditions (1) to (4) achieves an excellent usability such as post-application moisturizing effect, elastic feeling, and softness and that is excellent in emulsion stability while it is a composition which allows the makeup to stay on nicely because of its uniform applicability when used as a pre-makeup emulsion, thus leading to completion of the present invention.

That is, the water-in-oil emulsion composition of the present invention is characterized by comprising the below-described components (A) (B) and (C), and satisfies the below-described conditions (1) to (4).

### Components:

(A) 0.1 to 5.0 mass % of glycerol isostearate and/or glycerol oleate with respect to the total amount of composition,
(B) an aqueous component, and
(C) an oil component containing an isoparaffin having 20 carbon atoms or less and a solid oil component.

### Conditions:

(1) The internal-aqueous-phase proportion, which is obtained by dividing the mass of the aqueous component (B) by the sum of the mass of the aqueous component (B) and the mass of the oil component (C), is 68% or higher.
(2) The amount of glycerol monoisostearate and/or glycerol monooleate contained in component (A) is 85 mass % or higher with respect to the total amount of (A).
(3) The amount of an isoparaffine having 20 carbon atoms or less contained in component (C) is 30 mass % or higher with respect to the total amount of hydrocarbon oil.
(4) The phase equilibrium state obtained by mixing component (B) and component (A) is a multi-phase state, in which (i) bicontinuous cubic liquid crystals, reverse hexagonal liquid crystals, and a water phase are coexistent, or (ii) bicontinuous cubic liquid crystals, reverse hexagonal liquid crystals, other phases, and a water phase are coexistent.

It is preferable that the water-in-oil emulsion composition further comprises a component (D): tetramethyl trihydroxy hexadecane.

According to the invention, the water-in-oil emulsion composition further comprises a solid oil component as component (C).

### EFFECT OF THE INVENTION

A water-in-oil emulsion composition of the present invention comprises glycerol isostearate and/or glycerol oleate with less impurities, an aqueous component, an oil component containing an isoparaffin having 20 carbon atoms or less, and the present invention can provide a water-in-oil emulsion composition that provides a post-application elastic feeling and softness and that is excellent in emulsion stability while it is a composition with a high internal-aqueous-phase proportion, which allows the makeup to stay on nicely because of its uniform applicability when used as a pre-makeup emulsion.

### BEST MODE FOR CARRYING OUT THE INVENTION

A water-in-oil emulsion composition of the present invention comprises (A) glycerol isostearate and/or glycerol oleate (the amount of glycerol monoisostearate and/or glycerol monooleate is 85 mass % or higher), (B) an aqueous component, (C) an oil component containing an isoparaffin having 20 carbon atoms or less, and it meets that the internal-aqueous-phase proportion is 68% or higher. In the following, each component is described in detail.

### (A) Glycerol isostearate and/or glycerol oleate

The water-in-oil emulsion composition of the present invention is characterized by comprising glycerol isostearate and/or glycerol oleate (the amount of glycerol monoisostearate and/or glycerol monooleate is 85 mass % or higher) as surfactants. Among these, glycerol isostearate (the amount of glycerol monoisostearate is 85 mass % or higher) is preferably used in view of the smell.

Glycerol isostearate wherein glycerol monoisostearate has a high degree of purity can be obtained by various known synthesis methods. According to a common synthesis method, glycerol isostearate is formed as a mixture of glycerol monoisostearate, glycerol diisostearate, and glycerol triisostearate. It is necessary that the purity of glycerol monoisostearate contained in component (A) of the water-in-oil emulsion composition of the present invention is high (85 mass % or higher). A molecular distillation method is generally used as a method for purifying glycerol monoisostearate, however, the present invention is not limited thereto.

Glycerol oleate wherein glycerol monooleate has a high degree of purity can be obtained by various known synthesis methods. According to a common synthesis method, glycerol monooleate is formed as a mixture of glycerol monooleate, glycerol dioleate, and glycerol trioleate. It is necessary that the purity of glycerol monooleate contained in component (A) of the water-in-oil emulsion composition of the present invention is high. A molecular distillation method is generally used as a method for purifying glycerol monooleate, however, the present invention is not limited thereto.

It is necessary that the amount of glycerol monoisostearate and/or glycerol monooleate contained in component (A) is 85 mass % or higher with respect to the total amount of (A). That is to say, it is necessary that the amount of glycerol diisostearate and glycerol triisostearate and/or glycerol dioleate and glycerol trioleate, which are present as impurities of component (A), is 15 mass % or less with respect to the total amount of component (A). While glycerol monoisostearate and/or glycerol monooleate function as surfactants (i.e. emulsifiers), glycerol diisostearate and glycerol triisostearate and/or glycerol dioleate and glycerol trioleate behave as oils.

Accordingly, when purity of glycerol monoisostearate and/or glycerol monooleate is low and the amount of glycerol diisostearate and glycerol triisostearate and/or glycerol dioleate and glycerol trioleate, which are present as impurities of component (A) and/or contained in one of the oils in the oil components (C), exceeds 15 mass % with respect to the total amount of component (A), the emulsion stability may be deteriorated and the after-use light-refreshing feeling may be poor.

Purity of glycerol monoisostearate or glycerol monooleate can be measured with a common method such as gas chromatography (GC), gel permeation chromatography (GPC), and high-performance liquid chromatography (HPLC).

The amount of component (A) of the water-in-oil emulsion composition of the present invention is 0.1 to 5.0 mass % with respect to the total amount of the composition, more preferably 0.3 to 3.0 mass %, and particularly preferably 0.3 to 1.5 mass %. When the amount of component (A) is too small, the emulsion stability may be remarkably deteriorated. When it is too large, the usability such as after-use light-refreshing feeling may be poor.

### (B) Aqueous component

For component (B), the aqueous component, which can be generally used for cosmetics, pharmaceuticals, and so on, can be blended so far as it doesn't deteriorate emulsion stability.

Examples of moisturizers include 1,3-butylene glycol, polyethylene glycol, propylene glycol, dipropylene glycol, hexylene glycol, glycerin, diglycerin, xylitol, maltitol, maltose, and D-mannite.

Examples of water-soluble polymers include plant-based polymers such as gum arabic, carrageenan, pectine, agar, quince seed (marmelo), starch, and algae colloid (brown algae extract), microorganism-based polymers such as dextran and pullulan, animal-based polymers such as collagen, casein, and gelatine, starch-based polymers such as carboxymethyl starch and methylhydroxypropyl starch, alginic acid-based polymers such as sodium alginate, vinyl-based polymers such as carboxy vinyl polymer (e.g., CARBOPOL®), polyoxyethylene-based polymers, polyoxyethylene/polyoxypropylene copolymer-based polymers, acryl-based polymers such as sodium polyacrylate and polyacrylamide, and inorganic-based water-soluble polymers such as bentonite, magnesium aluminium silicate, and laponite.

Examples of UV absorbers include benzoic acid-based UV absorbers such as p-aminobenzoic acid, anthranilic acid-based UV absorbers such as methyl anthranilate, salicylic acid-based UV absorbers such as octyl salicylate and phenyl salicylate, cinnamic acid-based UV absorbers such as isopropyl p-methoxycinnamate, octyl p-methoxycinnamate, and glyceryl mono-2-ethylhexanoate di-p-methoxycinnamate, benzophenone-based UV absorbers such as 2,4-dihydroxybenzophenone, 2-hydroxy-4-methoxybenzophenone, and 2-hydroxy-4-methoxybenzophenone-5-sulfonic acid, urocanic acid, 2-(2'-hydroxy-5'-methylphenyl)benzotriazole, and 4-tert-butyl-4'-methoxybenzoylmethane.

Examples of sequestering agents include sodium edetate, sodium metaphosphate, and phosphoric acid.

Examples of antioxidants include ascorbic acid, alpha-tocopherol, dibutylhydroxytoluene, and butylhydroxyanisole.

Examples of drugs include vitamins such as vitamin A oil, retinol, retinol palmitate, inositol, pyridoxine hydrochloride, benzyl nicotinate, nicotinamide, dl-alpha-tocopherol nicotinate, magnesium ascorbyl phosphate, ascorbic acid 2-glucoside, vitamin D2 (ergocalciferol), L-ascorbic acid dl-alpha-tocopherol phosphoric acid diester potassium salt, dl-alpha-tocopherol, dl-alpha-tocopheryl acetate, pantothenic acid, and biotin, anti-inflammatory agents such as allantoin and azulene, whitening agents such as arbutin, astringent agents such as zinc oxide and tannic acid, sulfur, lysozyme chloride, pyridoxine hydrochloride, and gamma-orizanol.

The above-mentioned drugs can be used in a free state, a form of acid or basic salt if one can become salts, or a form of ester if one has a carboxylic acid group.

### (C) Oil component containing an isoparaffin having 20 carbon atoms or less

It is necessary that oil components (C) contain an isoparaffin having 20 carbon atoms or less. Examples of isoparaffins having 20 carbon atoms or less include isohexane (C₆H₁₄), isooctane (C₈H₁₈), isododecane (C₁₂H₂₆), and isohexadecane (C₁₆H₃₄). When a linear paraffin is blended, the effect of the present invention can't be obtained. In the present invention, it is preferable that the composition comprises isohexadecane and/or isododecane as the isoparaffin having 20 carbon atoms or less.

For component (C), in addition to the isoparaffin having 20 carbon atoms or less of the above-described essential component, the oil component, which can be generally used for cosmetics and pharmaceuticals, can be blended so far as it doesn't deteriorate emulsion stability.

Examples of liquid oils include silicone oils. Examples of silicone oils include liner silicone oils such as dimethylpolysiloxane, methylphenyl polysiloxane, and methylhydrogen polysiloxane, and cyclic silicone oils such as octamethylcyclotetrasiloxane, decamethylcyclopentasiloxane, and dodecamethylcyclohexasiloxane.

Examples of polar oils include ester oils such as cetyl octanoate, hexyl laurate, isopropyl myristate, octyl palmitate, isocetyl stearate, isopropyl isostearate, octyl isopalmitate, isodecyl isostearate, 2-ethylhexyl succinate, and diethyl sebacate.

Examples of non-polar oils include hydrocarbon oils such as liquid paraffin, squalane, squalene, and paraffin.

The mass of an isoparaffin having 20 carbon atoms or less contained in component (C) of the present invention is preferably 30 mass % or higher with respect to the total amount of hydrocarbon oil, particularly preferably 50 mass % or higher. When the mass of the isoparaffin is less than 30 mass % with respect to the total amount of hydrocarbon oil, the composition may lack after-use light-refreshing feeling.

According to the invention, the composition further comprises a solid oil component as the oil component of component (C). Examples of solid oil components include solid fats such as cacao butter, coconut oil, horse fat, hydrogenated coconut oil, palm oil, beef fat, mutton suet, and hydrogenated caster oil, hydrocarbons such as paraffin wax (linear hydrocarbon), microcrystalline wax (branched saturated hydrocarbon), ceresin wax, Japan wax, montan wax, and Fischer-Tropsch wax, waxes such as beeswax, lanolin, carnauba wax, candelilla wax, rice bran wax (rice wax), spermaceti, jojoba oil, insect wax, montan wax, kapok wax, bayberry wax, shellac wax, sugarcane wax, lanolin fatty acid isopropyl, hexyl laurate, reduced lanolin, hard lanolin, POE lanolin alcohol ether, POE lanolin alcohol acetate, POE cholesterol ether, lanolin fatty acid polyethylene glycol, and POE hydrogenated lanolin alcohol ether, higher fatty acids such as myristic acid, palmitic acid, stearic acid, and behenic acid, and higher alcohols such as cetyl alcohol, stearyl alcohol, behenyl alcohol, myristyl alcohol, and cetostearyl alcohols.

The amount of a solid oil component is preferably 0.5 to 3 mass % with respect to the total amount of the composition, more preferably 1 to 2 mass %. When the amount of solid oil component is less than 0.5 mass %, the moisturizing effect may be poor. When it exceeds 3 mass %, the spreadability on the skin may become heavy and the component may have an effect on the stability.

### (D) Tetramethyl trihydroxy hexadecane

In addition to the above-described components, it is preferable that the water-in-oil emulsion composition of the present invention comprises the nonionic surfactant, tetramethyl trihydroxy hexadecane. Examples of tetramethyl trihydroxy hexadecanes include Phytantriol (manufactured by DSM Nutritional Products) and etc. The water-in-oil emulsion composition, in which tetramethyl trihydroxy hexadecane is blended, can be better in temporal stability.

The internal-aqueous-phase proportion of the water-in-oil emulsion composition of the present invention is characterized to be 68% or higher. When the internal-aqueous-phase proportion is less than 68%, a light-refreshing feeling in use may not be obtained. In the present invention, the preparation of a water-in-oil emulsion composition having a high internal-aqueous-phase proportion of 80% or higher is possible, and a more light-refreshing feeling in use can be achieved. The internal-aqueous-phase proportion is calculated by dividing the mass of the aqueous component (B) by the sum of the mass of aqueous component (B) and the mass of oil component (C).

In the present invention, the preparation of a low-viscosity water-in-oil emulsion composition of 2000 to 15000 mPa·s is possible.

When the water-in-oil emulsion composition of the present invention is made less viscous, it is preferable that the mass of an isoparaffin having 20 carbon atoms or less is 30 mass % or higher with respect to the total amount of hydrocarbon oil and the blending quantity of the oil component is 15 mass % or higher with respect to the total amount of the composition. When the water-in-oil emulsion composition of the present invention is made less viscous, it is preferable that the internal-aqueous-phase proportion is 70 to 85% and it is more preferable that it is 75 to 85%.

In the past, it was difficult to prepare a low-viscosity water-in-oil emulsion composition.

In the case of an oil-in-water type emulsion, when the dispersed emulsion particles approach each other in the dispersion medium, the entropy repulsion of polyoxyethylene (POE) chains of nonionic surfactant around oil droplets and the electrostatic repulsion of ionic surfactant prevent coalescence. Accordingly, the instability by coalescence is of less concern even at a low viscosity.

On the other hand, when the most versatile POE type surfactant is selected as the emulsifying agent in a water-in-oil type emulsion, the lipophobicity of the hydrophilic POE group is low. Therefore, the surfactant that should adsorb to the interface is monodispersely dissolved in the oil phase and consumed by a large percentage. As a result, the efficient stabilization of the oil-water interface is difficult. Therefore, in the water-in-oil type emulsion, the oil that constitutes the outer phase is gelated and water droplets are immobilized. Thus, the stabilization is often attempted by decreasing the collision frequency of particles. That is, the achievement of both low viscosity and stability in the water-in-oil type emulsion has been difficult.

However, the water-in-oil emulsion composition of the present invention, in which the above-described components (A) to (C) are blended under specific conditions, can be a low-viscosity composition that is excellent in temporal emulsion stability.

It is known that there are four kinds of structures for cubic liquid crystals. There are discontinuous cubic liquid crystals in which the closed aggregate, micelles or reverse micelles, are cubically packed in the continuous oil or water layer, respectively, and there are bicontinuous cubic liquid crystals in which curved surfaces are formed by three-dimensionally connected lipid bilayers and cubically arranged to a bicontinuous structure. In the bicontinuous cubic liquid crystals, there is also a reversed type in which the locations of water and oil are reversed.

In the water-in-oil emulsion composition of the present invention, it is given that the phase equilibrium state obtained by mixing component (B) and component (A) is a multi-phase state, in which bicontinuous cubic liquid crystals, reverse hexagonal liquid crystals, and a water phase are coexistent, or bicontinuous cubic liquid crystals, reverse hexagonal liquid crystals, other phases, and a water phase are coexistent.

The water-in-oil emulsion composition having such phase equilibrium state allows applying uniformly to the skin. For example, when it is used as a pre-makeup emulsion, it allows the makeup to stay on nicely.

In bicontinuous cubic liquid crystals, bimolecular films of infinitely associated surfactant are cubically arranged. The appearance is transparent and optically isotropic, and they are a highly viscous gel. The discerning methods for bicontinuous cubic liquid crystals are the determination by appearance, preparation of a phase equilibrium diagram, measurement of electrical conductivity, measurement of a self-diffusion coefficient by NMR, small-angle X-ray scattering, and electron microscope observation of a replica prepared by the freeze-fracture method.

As the discerning method of the liquid crystal structure of the present invention, the following means can be considered. Initially, component (A), namely glycerol isostearate and/or glycerol oleate, and the aqueous component (B) are mixed well, and then coexistent plural phases are separated by a centrifugal treatment. When normal centrifugal equipment is used, the treatment time of several hours to several days may be necessary. If there is no coexistent phase and it is one phase state, the entirety will become uniformly transparent. In the bicontinuous cubic liquid crystals including the reverse type, the appearance is transparent and optically isotropic, and they are a highly viscous gel. Being optically isotropic can be recognized by the absence of transmitted light when a sample is retained between two polarizers that are combined at a phase difference of 90 degrees. The structure of the phase wherein the appearance is transparent and optically isotropic and it is a highly viscous gel, can be further identified by small-angle X-ray scattering. The scattering pattern for the bicontinuous cubic liquid crystals including the reverse type has a peak ratio of √2, √3, √4, √6, √8, √9 for the structure called Pn3m, or has a peak ratio of √6, √8, √14, √16, √20 for the structure called Ia3d.

As a simple method for the replacement of small-angle X-ray scattering, there is a structure estimation method, with the use of water-soluble and oil-soluble pigments, from the diffusion time as described in "H. Kunieda et al., J. Oleo Sci. vol. 52, 429-432 (2003)",.

The water-in-oil emulsion composition of the present invention can be widely applied for cosmetics, pharmaceuticals, and quasi-drugs which are commonly applied to the skin, and the specific examples include products such as a whitening essence, a milky lotion, a cream, a pack, a foundation, a lipstick, an eye shadow, a eyeliner, a mascara, a face wash, a spray, a mouse, a hair rinse, a shampoo, and a dermatological ointment.

### EXAMPLES

The present invention will be further described in the following examples, however, the invention is not limited by these examples. Unless otherwise specified, the blending quantity will be represented as mass % with respect to a system in which each component is blended.

Prior to illustrating the examples, the evaluation methods for the tests used in the present invention will be explained.

### Evaluation (1): After-use light-refreshing feeling

10 professional panelists applied each of the samples to face and evaluated the feeling in use during application.
A^{*}: Among 10 panelists, 9 or more panelists answered that the after-use light-refreshing feeling was present.
A: Among 10 panelists, 7 or more and less than 9 panelists answered that the after-use light-refreshing feeling was present.
B: Among 10 panelists, 5 or more and less than 7 panelists answered that the after-use light-refreshing feeling was present.
C: Among 10 panelists, less than 5 panelists answered that the after-use light-refreshing feeling was present.

### Evaluation (2): Elasticity

10 professional panelists applied each of the samples to face and evaluated the feeling in use during application.
A: Among 10 panelists, 8 or more panelists answered that the elasticity was present.
B: Among 10 panelists, 5 or more and less than 8 panelists answered that the elasticity was present.
C: Among 10 panelists, less than 5 panelists answered that the elasticity was present.

### Evaluation (3): Softness

10 professional panelists applied each of the samples to face and evaluated the feeling in use during application.
A: Among 10 panelists, 8 or more panelists answered that the softness was present.
B: Among 10 panelists, 5 or more and less than 8 panelists answered that the softness was present.
C: Among 10 panelists, less than 5 panelists answered that the softness was present.

### Evaluation (4): Moisturizing effect

10 professional panelists applied each of the samples to face and evaluated the feeling in use during application.
A^{*}: Among 10 panelists, 9 or more panelists answered that the moisturizing effect was present.
A: Among 10 panelists, 7 or more and less than 9 panelists answered that the moisturizing effect was present.
B: Among 10 panelists, 5 or more and less than 7 panelists answered that the moisturizing effect was present.
C: Among 10 panelists, less than 5 panelists answered that the moisturizing effect was present.

### Evaluation (5): Stability

The stability was evaluated by comparing the hardness and appearance of a sample stored for 1 month at 25 °C and 40 °C with those of a sample immediately after the preparation.
A^{*}: Under all storage conditions, the decrease of hardness was 10% or less, and no change in appearance was observed.
A: Under all storage conditions, no change in appearance was observed, and the decrease in the hardness of 10% or higher was observed only for the sample stored at 40 °C.
B^{*}: Under all storage conditions, no change in appearance was observed, and the decrease in the hardness of 10% or higher was observed.
B: The separation of water or oil was observed in the appearance.
C: Within 1 month, the separation of water or oil was observed in the appearance.

### Evaluation (6): Phase equilibrium when component (A) and component (B) were mixed

After mixing component (A) (surfactant) and component (B) adequately, each phase was separated by centrifugation. Then the phase equilibrium was determined by polarizing microscope observation and measurement of small-angle X-ray scattering.
A: Bicontinuous cubic liquid crystals, reverse hexagonal liquid crystals, and a water phase were coexistent.
B: Bicontinuous cubic liquid crystals, reverse hexagonal liquid crystals, other phases, and a water phase were coexistent.
C: The phase equilibrium consisted of the combination of phases other than bicontinuous cubic liquid crystals and reverse hexagonal liquid crystals (Liquid crystal phases weren't existent).

Initially, the present inventors prepared each sample with various blending quantities of oil components (internal-aqueous-phase proportion) with the blending composition shown in Table 1 in the below-described preparation method. Then, each sample was evaluated for the evaluation items (1) to (6) in the above-described evaluation criteria. The result is shown in Table 1.

The below-described internal-aqueous-phase proportion of the water-in-oil emulsion composition is calculated by dividing the mass of the aqueous component by the sum of the mass of the aqueous component and the mass of the oil component.

### Preparation method

Component (A), namely glycerol monoisostearate and/or glycerol monooleate (surfactants), and oil components (C) are mixed and heated to about 40 °C to be dissolved. Aqueous components (B) (and tetramethyl trihydroxy hexadecane (D)) are mixed and dissolved. After the water-soluble component part is gradually added to the oil-soluble component part while the oil-soluble component part is stirred relatively strongly (or heated), an emulsion is prepared. In some cases, an emulsion is prepared while both the oil-soluble component part and the water-soluble component part are heated. An emulsion is prepared by cooling the prepared emulsion with stirring when the prepared emulsion was heated.

**[Table 1]**

| Test Example | | 1-1 | 1-2 | 1-3 | 1-4 |
|---|---|---|---|---|---|
| (A) | Glycerol monoisostearate:85% | 1 | 1 | 1 | 1 |
| (B) | Glycerin | 5 | 5 | 5 | 5 |
| | Sodium chloride | 1 | 1 | 1 | 1 |
| | Ion-exchanged water | balance | balance | balance | balance |
| (C) | Squalane (C₃₀H₆₂) | 2 | 2 | 2 | 2 |
| | Decamethylcyclopentasiloxane | 36 | 26 | 18 | 8 |
| | Cetyl ethylhexanoate | 2.5 | 2.5 | 2.5 | 2.5 |
| Internal-aqueous-phase proportion | | 59% | 69% | 77% | 87% |
| Emulsion type | | W/O | W/O | W/O | W/O |
| Evaluation | (1) After-use light-refreshing feeling | B | B | A | A |
| | (2) Elasticity | B | B | B | B |
| | (3) Softness | B | B | B | B |
| | (4) Moisturizing effect | B | B | B | B |
| | (5) Stability | C | B | A | A |
| | (6) Phase equilibrium | B | A | A | A |

### Test Examples 1-1 to 1-4 are not covered by the scope of the invention.

As is clear from the results of Test Examples 1-3 and 1-4 (not covered by the scope of the invention), it has become possible for the samples, in which glycerol isostearate etc. are suitably blended, to retain the stability and provide an after-use light-refreshing feeling at the internal-aqueous-phase proportion of 70% or higher though it was difficult in the past, and it was confirmed that bicontinuous cubic liquid crystals, reverse hexagonal liquid crystals, and a water phase were coexistent. According to Test Example 1-2 (not covered by the scope of the invention), the coexistence of bicontinuous cubic liquid crystals, reverse hexagonal liquid crystals, and a water phase were confirmed to be possible at the internal-aqueous-phase proportion of 68% or higher.

As a result of the investigation by the present inventors, it was clarified that similar compositions to those of the above-described test examples, in which glycerol isostearate was used, could be obtained when glycerol oleate was used.

According to Table 1, however, the elasticity and softness and the moisturizing effect could not be improved at all even by increasing the internal-aqueous-phase proportion.

Next, the present inventors prepared each sample with various kinds and blending quantities of oil components based on the sample that can realize an after-use light-refreshing feeling at the high internal-aqueous-phase proportion (Test Example 1-4). Each sample was prepared with the blending composition shown in Table 2 in the above-described preparation method. Then, each sample was evaluated for the evaluation items (1) to (6) in the above-described evaluation criteria. The result is shown in Table 2. Test Examples 2-1 to 2-10 are not covered by the scope of the invention.

**[Table 2]**

| Test Example | | 1-4 | 2-1 | 2-2 | 2-3 | 2-4 | 2-5 | 2-6 | 2-7 | 2-8 | 2-9 | 2-10 |
|---|---|---|---|---|---|---|---|---|---|---|---|---|
| (A) | Glycerol monoisostearate:85% | 1 | 1 | 1 | 1 | 0.7 | 0.7 | 0.7 | 0.7 | 0.7 | 1 | 0.7 |
| (B) | Glycerin | 5 | 5 | 5 | 5 | 5 | 5 | 5 | 5 | 5 | 5 | 5 |
| | Sodium chloride | 1 | 1 | 1 | 1 | 1 | 1 | 1 | 1 | 1 | 1 | 1 |
| | Ion-exchanged water | balance | balance | balance | balance | balance | balance | balance | balance | balance | balance | balance |
| (C) | Squalane (C₃₀H₆₂) | 2 | 2 | 2 | 2 | 2 | 5 | 7 | 9 | 10 | - | 2 |
| | Isohexadecane (C₁₆H₃₄) | - | 8 | - | - | - | 5 | 3 | 1 | - | 3 | - |
| | - Isododecane (C₁₂H₂₆) | - | - | 8 | 12 | 8 | - | - | - | - | - | - |
| | The proportion of an isoparaffin having 20 carbon atoms or less with respect to the total amount of hydrocarbon oil | 0% | 80% | 80% | 86% | 80% | 50% | 30% | 10% | 0% | 100% | 0% |
| | Cetyl ethylhexanoate | 2.5 | 2.5 | 2.5 | 2.5 | 2.5 | 2.5 | 2.5 | 2.5 | 2.5 | 2.5 | 2.5 |
| | Decamethylcyclopentasiloxane | 8 | - | - | - | - | - | - | - | - | 7 | - |
| | Liquid paraffin | - | - | - | - | - | - | - | - | - | - | 8 |
| (D) | Tetramethyl trihydroxy hexadecane 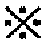 1 | - | - | - | - | 0.3 | 0.3 | 0.3 | 0.3 | 0.3 | - | 0.3 |
| Internal-aqueous-phase proportion | | 87% | 87% | 87% | 83% | 87% | 87% | 87% | 87% | 87% | 87% | 87% |
| Emulsion type | | W/O | W/O | W/O | W/O | W/O | W/O | W/O | W/O | W/O | W/O | W/O |
| Evaluation | (1) After-use light-refreshing feeling | A | A* | A* | A* | A* | A* | A | B | B | A* | B |
| | (2) Elasticity | B | A | A | A | A | A | A | A | A | A | A |
| | (3) Softness | B | A | A | A | A | A | A | A | A | A | A |
| | (4) Moisturizing effect | B | A | A | A | A | A | A | A | A | A | A |
| | (5) Stability | A | A | A | A* | A* | A* | A* | A* | A* | A* | A* |
| | (6) Phase equilibrium | A | A | A | A | A | A | A | A | A | A | A |

| | | | | | | | | | | | | |
|---|---|---|---|---|---|---|---|---|---|---|---|---|
| 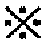 1: Phytantriol (manufactured by DSM Nutritional Products) | | | | | | | | | | | | |

The samples of Test Examples 2-1 and 2-2 (not covered by the scope of the invention), in which a short-chain isoparaffin (isohexadecane or isododecane) was blended instead of decamethylcyclopentasiloxane in Test Example 1-4, were better in the after-use light-refreshing feeling. In addition, the post-application elastic feeling and softness and the moisturizing effect could be achieved. When the blending quantity of the short-chain isoparaffin was increased, the stability was found to improve further (Test Example 2-3, not covered by the scope of the invention).

As a result of further investigation by the present inventors, it was clarified that when a short-chain isoparaffin, in particular, an isoparaffin having 20 carbon atoms or less was blended as the oil component, the post-application elastic feeling and softness and the moisturizing effect could be provided to the water-in-oil emulsion composition having a high internal-aqueous-phase proportion.

In addition, the sample of Test Example 2-4 (not covered by the scope of the invention), in which tetramethyl trihydroxy hexadecane was blended as the additional surfactant, was excellent in usability, and the stability was also very good.

On the other hand, when the blending quantity of a long-chain paraffin (squalane) was gradually increased and the blending quantity of an isoparaffin having 20 carbon atoms or less was decreased with respect to the total amount of hydrocarbon oil (Test Examples 2-5 to 2-8; not covered by the scope of the invention), the post-application elastic feeling and softness could be maintained; however, the after-use light-refreshing feeling was lost gradually.

In Test Example 2-9 (not covered by the scope of the invention), in which the composition of Test Example 2-6 was somewhat varied and the percentage of an isoparaffin having 20 carbon atoms or less was increased with respect to the total amount of hydrocarbon oil, an excellent after-use light-refreshing feeling could be achieved compared with Test

### Example 2-6.

The sample of Test Example 2-10 (not covered by the scope of the invention), in which a liquid paraffin was blended instead of isododecane in Test Example 2-4, was poor in the after-use light-refreshing feeling similar to Test Example 2-8.

Accordingly, it was clarified that the blending quantity of an isoparaffin having 20 carbon atoms or less is preferably 30 mass % or higher with respect to the total amount of hydrocarbon oil.

Next, the present inventors studied the blending quantity of component (A).

The present inventors prepared each sample with various blending quantities of glycerol isostearate as shown in Table 3 in the above-described preparation method. Then, each sample was evaluated for the evaluation items (1) to (6) in the above-described evaluation criteria. The result is shown in Table 3. Test Examples 2-1 to 2-10 are not covered by the scope of the invention.

**[Table 3]**

| Test Example | | 3-1 | 3-2 | 3-3 | 3-4 | 3-5 |
|---|---|---|---|---|---|---|
| (A) | Glycerol monoisostearate:85% | 0.05 | 0.3 | 1.5 | 3.0 | 6.0 |
| (B) | Glycerin | 5 | 5 | 5 | 5 | 5 |
| | Sodium chloride | 1 | 1 | 1 | 1 | 1 |
| | Ion-exchanged water | balance | balance | balance | balance | balance |
| (C) | Squalane | 7 | 7 | 7 | 7 | 7 |
| | Isododecane | 3 | 3 | 3 | 3 | 3 |
| | Cetyl ethylhexanoate | 2.5 | 2.5 | 2.5 | 2.5 | 2.5 |
| (D) | Tetramethyl trihydroxy hexadecane 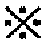 1 | 0.3 | 0.3 | 0.3 | 0.3 | 0.3 |
| Emulsion type | | W/O | W/O | W/O | W/O | W/O |
| Evaluation | (1) After-use light-refreshing feeling | A* | A* | A | A | C |
| | (2) Elasticity | A | A | A | A | A |
| | (3) Softness | A | A | A | A | A |
| | (4) Moisturizing effect | A | A | A | A | A |
| | (5) Stability | C | A* | A* | A* | A |
| | (6) Phase equilibrium | C | A | A | A | A |

| | | | | | | |
|---|---|---|---|---|---|---|
| 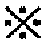 1: Phytantriol (manufactured by DSM Nutritional Products) Test Examples 3-1 to 3-5 are not covered by the scope of the invention. | | | | | | |

According to Table 3, the phase equilibrium of the sample in Test Example 3-1 (not covered by the scope of the invention), in which 0.05 mass % of component (A) was blended, consists of phases other than bicontinuous cubic liquid crystals and reverse hexagonal liquid crystals, and the emulsion stability tended to be poor. On the other hand, when component (A) was suitably blended (Test Examples 3-2 to 3-5; not covered by the scope of the invention), bicontinuous cubic liquid crystals, reverse hexagonal liquid crystals, and a water phase were coexistent, and it was found that the excellent emulsion stability could be obtained. However, when 6.0 mass % of component (A) was blended (Test Example 3-5), the after-use light-refreshing feeling was poor.

Accordingly, it was clarified that the blending quantity of component (A) in the water-in-oil emulsion composition of the present invention is 0.1 to 5.0 mass %.

Next, the present inventors studied the evaluation of phase state, emulsion stability, and usability of the water-in-oil emulsion composition having a high internal-aqueous-phase proportion.

The present inventors prepared each sample comprising glycerol monoisostearate with various purities with the blending composition shown in Table 4 in the above-described preparation method. Then, each sample was evaluated for the evaluation items (1) to (6) in the above-described evaluation criteria. The result is shown in Table 4. Test Examples 4-1 to 4-8 are not covered by the scope of the invention.

**[Table 4]**

| Test Example | | 4-1 | 4-2 | 4-3 | 4-4 | 4-5 | 4-6 | 4-7 | 4-8 | 3-2 |
|---|---|---|---|---|---|---|---|---|---|---|
| (A) | Glycerol monoisostearate:95% | 0.7 | - | - | - | - | - | - | - | - |
| | Glycerol monoisostearate:85% | - | 0.7 | - | - | - | - | - | - | 0.3 |
| | Glycerol monoisostearate:70% | - | - | 0.7 | - | - | - | - | - | - |
| | Glycerol monoisostearate:45% | - | - | - | 0.7 | - | - | - | - | - |
| | Glycerol monoisostearate:100% | - | - | - | - | 0.67 | 0.6 | 0.49 | 0.32 | - |
| (C) | Glycerol diisostearate | - | - | - | - | 0.03 | 0.07 | 0.15 | 0.27 | - |
| | Glycerol triisostearate | - | - | - | - | - | 0.03 | 0.06 | 0.11 | - |
| | The proportion of impurities | 5% | 15% | 30% | 55% | 4% | 14% | 30% | 54% | 15% |
| (B) | Glycerin | 5 | 5 | 5 | 5 | 5 | 5 | 5 | 5 | 5 |
| | Sodium chloride | 1 | 1 | 1 | 1 | 1 | 1 | 1 | 1 | 1 |
| | Ion-exchanged water | balance | balance | balance | balance | balance | balance | balance | balance | balance |
| (C) | Squalane | 7 | 7 | 7 | 7 | 7 | 7 | 7 | 7 | 7 |
| | Isododecane | 3 | 3 | 3 | 3 | 3 | 3 | 3 | 3 | 3 |
| | Cetyl ethylhexanoate | 2.5 | 2.5 | 2.5 | 2.5 | 2.5 | 2.5 | 2.5 | 2.5 | 2.5 |
| (D) | Tetramethyl trihydroxy hexadecane 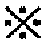 1 | 0.3 | 0.3 | 0.3 | 0.3 | 0.3 | 0.3 | 0.3 | 0.3 | 0.3 |
| Emulsion type | | W/O | W/O | W/O | W/O | W/O | W/O | W/O | W/O | W/O |
| Evaluation | (1) After-use light-refreshing feeling | A | A | B | C | A | A | B | C | A* |
| | (2) Elasticity | A | A | A | A | A | A | A | A | A |
| | (3) Softness | A | A | A | A | A | A | A | A | A |
| | (4) Moisturizing effect | A | A | A | A | A | A | A | A | A |
| | (5) Stability | A* | A* | B | C | A* | A* | B | C | A* |
| | (6) Phase equilibrium | A | A | B | C | A | A | B | C | A |

| | | | | | | | | | | |
|---|---|---|---|---|---|---|---|---|---|---|
| 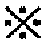 1: Phytantriol (manufactured by DSM Nutritional Products) | | | | | | | | | | |

According to Table 4, when the purity of glycerol monoisostearate was 95% and 85% (Test Examples 4-1 and 4-2; not covered by the scope of the invention), the compositions excellent in both emulsion stability and usability were obtained.

When the purity was 70% (Test Example 4-3; not covered by the scope of the invention), however, in addition to bicontinuous cubic liquid crystals, reverse hexagonal liquid crystals, and a water phase, other phases were coexistent in the phase equilibrium. The emulsion stability at a low temperature was somewhat lower, and the after-use light-refreshing feeling was not satisfactory. When the purity was further lowered to 45% (Test Example 4-4; not covered by the scope of the invention), the phase equilibrium consisted of phases other than bicontinuous cubic liquid crystals and reverse hexagonal liquid crystals. The emulsion stability and the after-use light-refreshing feeling also had a trend to decrease further.

The samples of Test Examples 4-5 to 4-8 (not covered by the scope of the invention), in which glycerol monoisostearate without impurities, glycerol diisostearate, and glycerol triisostearate were blended, were evaluated to have a similar phase equilibrium and usability to those of the samples of Test Examples 4-1 to 4-4, respectively, in which about the same percentage of impurities are contained.

When Test Example 4-8 and Test Example 3-2 are compared, regardless of approximately the same blending quantity of glycerol monoisostearate, the sample of Test Example 3-2, in which the purity of glycerol monoisostearate is high and the blending quantities of glycerol diisostearate and glycerol triisostearate are small, was much better in the after-use light-refreshing feeling and stability.

Accordingly, when the purity decreases (di-form and tri-form increase), the stability deteriorates, the oily feeling increases, and the after-use light-refreshing feeling is lost.

From the above, in the water-in-oil emulsion composition of the present invention, it is necessary that the amount of glycerol monoisostearate and/or glycerol monooleate contained in component (A) is 85 mass % or higher with respect to the total amount of (A). That is to say, it is necessary that the amount of glycerol diisostearate and glycerol triisostearate and/or glycerol dioleate and glycerol trioleate, which are present as impurities of component (A) and/or contained one of the oil components (C), is 15 mass % or less with respect to the total amount of component (A).

Next, the present inventors studied another oil components.

The present inventors prepared each sample with various kinds of another oil components as shown in Table 5 in the above-described preparation method. Then, each sample was evaluated for the evaluation items (1) to (6) in the above-described evaluation criteria. The result is shown in Table 5. Test Example 5-1 is not covered by the scope of the invention.

**[Table 5]**

| Test Example | | 5-1 | 5-2 | 5-3 | 5-4 | 5-5 | 5-6 |
|---|---|---|---|---|---|---|---|
| (A) | Glycerol monoisostearate:85% | 0.7 | 0.7 | 0.7 | 0.7 | - | - |
| | Glycerol monooleate:90% | - | - | - | - | 0.7 | 0.7 |
| (B) | Glycerin | 5 | 5 | 5 | 5 | 5 | 5 |
| | Sodium chloride | 1 | 1 | 1 | 1 | 1 | 1 |
| | Ion-exchanged water | balance | balance | balance | balance | balance | balance |
| (C) | Isohexadecane | 7 | 7 | 7 | 7 | 7 | 7 |
| | Cetyl ethylhexanoate | 2.5 | 2.5 | 2.5 | 2.5 | 2.5 | 2.5 |
| | Petrolatum | - | 2 | 2 | 2 | 2 | 2 |
| | (Paraffin/microcrystalline wax) mixture | - | 1 | 2 | 4 | 2 | 4 |
| (D) | Tetramethyl trihydroxy hexadecane 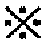 1 | 0.3 | 0.3 | 0.3 | 0.3 | 0.3 | 0.3 |
| Emulsion type | | W/O | W/O | W/O | W/O | W/O | W/O |
| Evaluation | (1) After-use light-refreshing feeling | A | A | A | B | A | B |
| | (2) Elasticity | A | A | A | A | A | A |
| | (3) Softness | A | A | A | A | A | A |
| | (4) Moisturizing effect | A | A* | A* | A* | A* | A* |
| | (5) Stability | A* | A* | A* | B | A* | B |
| | (6) Phase equilibrium | A | A | A | A | A | A |

| | | | | | | | |
|---|---|---|---|---|---|---|---|
| 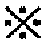 1: Phytantriol (manufactured by DSM Nutritional Products) | | | | | | | |

In addition to cetyl ethylhexanoate, which is a liquid oil component, the addition of petrolatum, which is a semisolid oil component, and a paraffin/microcrystalline wax mixture, which is a solid oil component, was investigated. As a result, Test Examples 5-2 and 5-3, in which 1 mass % and 2 mass % of the solid oil component were blended, respectively, had both additional elasticity and softness because squalane was not used as the liquid hydrocarbon oil and only isohexadecane was blended. Furthermore, by the addition of the solid oil component, a better moisturizing effect was achieved. On the other hand, in Test Example 5-4, in which 4 mass % of the solid oil component was blended, no change was observed in the quality of the moisturizing effect; however, the after-use light-refreshing feeling and the stability became worse.

Test Example 5-5, in which glycerol monooleate was blended instead of glycerol monoisostearate in Test Example 5-3, was confirmed to have a similar evaluation to Test Examples 5-3 as the above results.

Test Example 5-6, in which glycerol monooleate was blended instead of glycerol monoisostearate in Test Example 5-4, was also confirmed to have a similar evaluation to Test Examples 5-4.

Accordingly, in the water-in-oil emulsion composition of the present invention, in view of moisuturizing effect, it is preferable that the composition comprises 0.5 to 3 mass % of a solid oil component with respect to the total amount of composition.

Next, the present inventors studied whether the composition of low viscosity was available.

The present inventors prepared each sample with various kinds of surfactants such as component (A) and various kinds and blending quantities of oil components. Each sample was prepared as shown in Table 6 in the above-described preparation method. Then, each sample was evaluated for the evaluation items (5) and (6) in the above-described evaluation criteria. Each sample was also evaluated for the evaluation items (7) and (8) in the below-described evaluation criteria. The result is shown in Table 6.

### Evaluation (7): Low viscosity

Each sample was stored in a screw vial and evaluated its state.
A: The sample is stored in a screw vial; when the screw vial is tilted to the side, the emulsion flows immediately.
A: The sample is stored in a screw vial; when the screw vial is tilted to the side, the emulsion flows slowly.
B: The sample is stored in a screw vial; when the screw vial is turned upside down, the emulsion flows slowly.
C: The sample is stored in a screw vial; even when the screw vial is turned upside down, the emulsion does not flow.

### Evaluation (8): Viscosity

The viscosity of each sample one hour after the preparation (stored at 30 °C) was measured with a B-type viscometer (BL-type, 12 rpm).

**[Table 6]**

| Test Example | | 6-1 | 6-2 | 6-3 | 6-4 | 6-5 | 6-6 | 6-7 | 6-8 | 6-9 |
|---|---|---|---|---|---|---|---|---|---|---|
| (A) | Glycerol monoisostearate:85% | 0.5 | 0.5 | 0.5 | 0.5 | 0.5 | 0.5 | 0.5 | 0.5 | - |
| | Glycerol monooleate:90% | - | - | - | - | - | - | - | - | 0.5 |
| (B) | Glycerin | 5 | 5 | 5 | 5 | 5 | 5 | 5 | 5 | 5 |
| | Sodium chloride | 1 | 1 | 1 | 1 | 1 | 1 | 1 | 1 | 1 |
| | Ion-exchanged water | balance | balance | balance | balance | balance | balance | balance | balance | balance |
| (C) | Squalane (C₃₀H₆₂) | 42 | 29 | 18 | 10 | - | - | - | - | - |
| | Isohexadecane (C₁₆H₃₄) | - | - | - | - | 42 | 29 | 18 | 10 | 18 |
| | The proportion of an isoparaffin having 20 carbon atoms or less with respect to the total amount of hydrocarbon oil | 0% | 0% | 0% | 0% | 100% | 100% | 100% | 100% | 100% |
| | Cetyl ethylhexanoate | 2 | 2 | 2 | 2 | 2 | 2 | 2 | 2 | 2 |
| Internal-aqueous-phase proportion | | 56% | 69% | 80% | 88% | 56% | 69% | 80% | 88% | 80% |
| Emulsion type | | W/O | W/O | W/O | W/O | W/O | W/O | W/O | W/O | W/O |
| Evaluation | (5) Stability | C | B | A | A* | B | A | A* | A* | A* |
| | (6) Phase equilibrium | A | A | A | A | A | A | A | A | A |
| | (7) Low viscosity | A* | A | B | C | A* | A* | A* | B | A* |
| | (8) Viscosity (mPa·s) | - | 14800 | 24200 | 43500 | 2100 | 3520 | 7510 | 21000 | 9230 |

### Test examples 6-1 to 6-9 are not covered by the scope of the invention.

According to Table 6, when squalane was mainly blended as the oil component, as in Test Examples 6-1 to 6-4 (not covered by the scope of the invention), the viscosity decreased with an increase in the amount of the oil component; however, the stability became worse. In the samples with an internal-aqueous-phase proportion of 68% or higher, as in Test Examples 6-2 to 6-4, the viscosity was high, and it became creamy, less fluid, but more stable with an increase in the internal-aqueous-phase proportion.

As shown in Test Examples 6-5 to 6-8 (not covered by the scope of the invention), when isohexadecane was blended in the sample, low-viscosity stable compositions could be prepared. However, when the blending quantity of the oil component was very small, as in Test Example 6-8, the achievement of low viscosity was difficult. Accordingly, when the composition is made less viscous, the blending quantity of oil component is preferably 15 mass % or higher.

As shown in Test Example 6-9 (not covered by the scope of the invention), when glycerol monooleate was used as surfactant instead of glycerol monoisostearate, lowly viscous and stable composition could be prepared.

### The present inventors further studied low viscosity of the composition.

The present inventors prepared each sample with various kinds and blending quantities of oil components as shown in Table 7 in the above-described preparation method. Then, each sample was evaluated for the evaluation items (5) to (8) in the above-described evaluation criteria. The result is shown in Table 7. Test Examples 7-1 to 7-10 are not covered by the scope of the invention)

**[Table 7]**

| Test Example | | 7-1 | 7-2 | 7-3 | 7-4 | 7-5 | 7-6 | 7-7 | 7-8 | 7-9 | 7-10 |
|---|---|---|---|---|---|---|---|---|---|---|---|
| (A) | Glycerol monoisostearate:85% | 0.5 | 0.5 | 0.5 | 0.5 | 0.5 | 0.5 | - | - | - | - |
| | Polyglyceryl diisostearate | - | - | - | - | - | - | 2 | 2 | - | - |
| | Alkyl/ polyether-comodified silicone 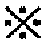 2 | - | - | - | - | - | - | - | - | 2 | 2 |
| (B) | Glycerin | 5 | 5 | 5 | 5 | 5 | 5 | 5 | 5 | 5 | 5 |
| | Sodium chloride | 1 | 1 | 1 | 1 | 1 | 1 | 1 | 1 | 1 | 1 |
| | Ion-exchanged water | balance | balance | balance | balance | balance | balance | balance | balance | balance | balance |
| (C) | Squalane (C₃₀H₆₂) | 9 | 12 | 16 | - | 10 | - | 18 | - | 10 | - |
| | Isohexadecane (C₁₆H₃₄) | 9 | 6 | 2 | - | - | 10 | - | 18 | - | 10 |
| | The proportion of an isoparaffin having 20 carbon atoms or less with respect to the total amount of hydrocarbon oil | 50% | 33% | 11% | 0% | 0% | 100% | 0% | 100% | 0% | 100% |
| | Cetyl ethylhexanoate | 2 | 2 | 2 | - | - | - | 2 | 2 | 6 | 6 |
| | Decamethylcyclopentasiloxane | - | - | - | 20 | 10 | 10 | - | - | 4 | 4 |
| Internal-aqueous-phase proportion | | 80% | 80% | 80% | 80% | 80% | 80% | 80% | 80% | 80% | 80% |
| Emulsion type | | W/O | W/O | W/O | W/O | W/O | W/O | W/O | W/O | W/O | W/O |
| Evaluation | (5) Stability | A* | A* | A* | B | A | A | C | C | A | A |
| | (6) Phase equilibrium | A | A | A | A | A | A | C | C | C | C |
| | (7) Low viscosity | A* | A | B | B | B | A* | A* | A* | C | C |
| | (8) Viscosity (mPa·s) | 10500 | 16050 | 23100 | - | 21400 | 7980 | - | - | - | - |

| | | | | | | | | | | | |
|---|---|---|---|---|---|---|---|---|---|---|---|
| 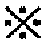 2: ABIL EM 90 (manufactured by Evonik Degussa Japan Co., Ltd.) | | | | | | | | | | | |

As shown in Test Examples 7-1 to 7-3 (not covered by the scope of the invention), when the blending quantity of the long-chain paraffin (squalane) was gradually increased and the percentage of the isoparaffin having 20 carbon atoms or less (isohexadecane) was decreased with respect to the hydrocarbon oil, the achievement of low-viscosity tends to be poorer. Accordingly, it was confirmed that if the amount of an isoparaffin having 20 carbon atoms or less is 30% or higher with respect to the total amount of hydrocarbon oil, both the stability and low viscosity could be achieved.

Likewise, Test Examples 7-4 to 7-6 (not covered by the scope of the invention), in which decamethylcyclopentasiloxane was used as one of the oil components, could achieve low viscosity when the large amount of an isoparaffin having 20 carbon atoms or less was blended.

On the other hand, the sample of Test Example 7-4, in which decamethylcyclopentasiloxane was blended but isoparaffin having 20 carbon atoms wasn't blended, was a gel and could not achieve low viscosity. Test Example 7-5, in which a part of decamethylcyclopentasiloxane was replaced with squalane, became better in the stability compared with Test Example 7-4; however, the viscosity was high.

As shown in Test Examples 7-7 and 7-8 (not covered by the scope of the invention), when polyglyceryl diisostearate was used as the surfactant, the viscosity was very low; however, the stability became worse.

As shown in Test Examples 7-9 and 7-10 (not covered by the scope of the invention), when alkyl/polyether-comodified silicone was used as the surfactant, the stability was excellent; however, low viscosity could not be achieved.

From the above, it was clarified that the water-in-oil emulsion composition of the present invention, in which components (A) to (C) are blended under specific conditions, can be a low-viscosity composition while it is excellent in temporal emulsion stability and usability.

When the composition is made less viscous, it is preferable that the mass of an isoparaffin having 20 carbon atoms or less is 30 mass % or higher with respect to the total amount of hydrocarbon oil and the blending quantity of the oil component is 15 mass % or higher with respect to the total amount of the composition.

Hereinafter, formulation examples of the water-in-oil emulsion composition of the present invention will be illustrated. It is to be understood that the present invention is not limited by these formulation examples.

### Formulation Example 1: Moisturizing cream

| (Blending components) | (mass %) |
|---|---|
| (1) Purified water | balance |
| (2) Sodium chloride | 1.0 |
| (3) Glycerin | 5.0 |
| (4) Glycerol isostearate (pure component of glycerol monoisostearate: 85 mass %) | 0.7 |
| (5) Squalane | 2.0 |
| (6) Isohexadecane | 8.0 |
| (7) Cetyl ethylhexanoate | 2.5 |
| (8) Petrolatum | 2.0 |
| (9) (Paraffin/microcrystalline wax) mixture | 1.0 |
| (10) Tetramethyl trihydroxy hexadecane | 0.3 |
| (11) Perfume | proper quantity |

The proportion of an isoparaffin having 20 carbon atoms or less with respect to the total amount of hydrocarbon oil: 80.0%

The internal-aqueous-phase proportion: 85.3%

### (Process and evaluation)

The oil components (4) to (11) were mixed, and the uniform dispersion of the oil phase was carried out with heating. The water phase containing components (1) to (3) were mixed. The water phase with heating gradually added to the oil phase. After the dispersion with a homodisper to uniform emulsion particles and the cooling with stirring, a moisturizing cream of the water-in-oil emulsion composition was prepared. The stability of the obtained moisturizing cream was excellent and the cream had both post-application elastic feeling and softness in addition to after-use light-refreshing feeling.

### Formulation Example 2: Moisturizing cream

| (Blending components) | (mass %) |
|---|---|
| (1) Purified water | balance |
| (2) Sodium chloride | 1.0 |
| (3) Glycerin | 5.0 |
| (4) Glycerol isostearate (pure component of glycerol monoisostearate: 85 mass %) | 0.7 |
| (5) Squalane | 2.0 |
| (6) Isohexadecane | 5.0 |
| (7) Tridecyl isononanoate | 2.5 |
| (8) Petrolatum | 2.0 |
| (9) (Polyethylene 25%/microcrystalline wax 75%) mixture | 1.0 |
| (10) Tetramethyl trihydroxy hexadecane | 0.3 |
| (11) Perfume | proper quantity |
| (12) Potassium sulfite | 0.005 |

The proportion of an isoparaffin having 20 carbon atoms or less with respect to the total amount of hydrocarbon oil: 71.4%

The internal-aqueous-phase proportion: 88.4%

### (Process and evaluation)

The oil components (5) to (12) were mixed at room temperature, and the uniform dispersion of the oil phase was carried out. The water phase containing components (1) to (4) were gradually added to the oil phase. After the dispersion with a homodisper to uniform emulsion particles, a moisturizing cream of the water-in-oil emulsion composition was prepared. The stability test of the obtained moisturizing cream was carried out in the same way as described above. The stability of the obtained moisturizing cream was excellent, there was no strange smell at high temperatures over time, and the cream had good usability with excellent tautness (in other words, firmness, tension, elasticity, resilience, and suppleness).

### Formulation Example 3: Water-in-oil pre-makeup emulsion

| (Blending components) | (mass %) |
|---|---|
| (1) Purified water | balance |
| (2) Sodium chloride | 1.0 |
| (3) Glycerin | 5.0 |
| (4) Glycerol isostearate (pure component of glycerol monoisostearate: 85 mass %) | 0.7 |
| (5) Squalane | 2.0 |
| (6) Isohexadecane | 7.0 |
| (7) Isononyl isononanoate | 2.5 |
| (8) Petrolatum | 2.0 |
| (9) (Paraffin 92.5%/microcrystalline wax 7.5%) mixture | 1.0 |
| (10) Tetramethyl trihydroxy hexadecane | 0.3 |
| (11) Perfume | proper quantity |
| (12) Sodium pyrosulfite | 0.2 |
| (13) Talc | 2.0 |

The proportion of an isoparaffin having 20 carbon atoms or less with respect to the total amount of hydrocarbon oil: 77.8%

The internal-aqueous-phase proportion: 86.4%

### (Process and evaluation)

The oil components (5) to (12) were mixed at room temperature, and the uniform dispersion of the oil phase was carried out. The water phase containing components (1) to (4), and (13) were gradually added to the oil phase. After the dispersion with a homodisper to uniform emulsion particles, a water-in-oil pre-makeup emulsion of the water-in-oil emulsion composition was prepared. The stability test of the obtained water-in-oil pre-makeup emulsion was carried out in the same way as described above. The stability of the obtained water-in-oil pre-makeup emulsion was excellent and the cream had good usability with excellent tautness (in other words, firmness, tension, elasticity, resilience, and suppleness).

### Formulation Example 4: Moisturizing cream

| (Blending components) | (mass %) |
|---|---|
| (1) Purified water | balance |
| (2) Sodium chloride | 1.0 |
| (3) Glycerin | 5.0 |
| (4) Glycerol isostearate (pure component of glycerol monoisostearate: 85 mass %) | 0.4 |
| (5) Glycerol monooleate (pure component of glycerol monooleate: 90 mass %) | 0.3 |
| (6) Squalane | 2.0 |
| (7) Isohexadecane | 5.0 |
| (8) Tridecyl isononanoate | 2.5 |
| (9) Petrolatum | 2.0 |
| (10) (Polyethylene 25%/microcrystalline wax 75%) mixture | 1.0 |
| (11) Tetramethyl trihydroxy hexadecane | 0.3 |
| (12) Perfume | proper quantity |
| (13) Potassium sulfite | 0.005 |

The proportion of an isoparaffin having 20 carbon atoms or less with respect to the total amount of hydrocarbon oil: 71.4%

The internal-aqueous-phase proportion: 88.4%

### (Process and evaluation)

The oil components (6) to (13) were mixed at room temperature, and the uniform dispersion of the oil phase was carried out. The water phase containing components (1) to (5) were gradually added to the oil phase. After the dispersion with a homodisper to uniform emulsion particles, a moisturizing cream of the water-in-oil emulsion composition was prepared. The stability test of the obtained moisturizing cream was carried out in the same way as described above. The stability of the obtained moisturizing cream was excellent, there was no strange smell at high temperatures over time, and the cream had good usability with excellent tautness (in other words, firmness, tension, elasticity, resilience, and suppleness).

## Claims

1. A water-in-oil emulsion composition comprising the below-described components (A) (B) and (C), wherein it satisfies the below-described conditions (1) to (4);
Components:
(A) 0.1 to 5.0 mass % of glycerol isostearate and/or glycerol oleate with respect to the total amount of composition,
(B) an aqueous component, and
(C) an oil component containing an isoparaffin having 20 carbon atoms or less and a solid oil component;
Conditions:
(1) the internal-aqueous-phase proportion, which is obtained by dividing the mass of the aqueous component (B) by the sum of the mass of the aqueous component (B) and the mass of the oil component (C), is 68% or higher, and
(2) the amount of glycerol monoisostearate and/or glycerol monooleate contained in component (A) is 85 mass % or higher with respect to the total amount of (A),
(3) the amount of an isoparaffin having 20 carbon atoms or less contained in component (C) is 30 mass % or higher with respect to the total amount of hydrocarbon oil,
and
(4) in the water-in-oil emulsion component (B) and component (A) are present in a multi-phase equilibrium state, wherein the multi-phase equilibrium state is obtained by mixing component (B) and component (A), and in which the multi-phase equilibrium state is (i) bicontinuous cubic liquid crystals, reverse hexagonal liquid crystals, and a water phase are coexistent, or (ii) bicontinuous cubic liquid crystals, reverse hexagonal liquid crystals, other phases, and a water phase are coexistent.

2. The water-in-oil emulsion composition according to claim 1, further comprising a component (D): tetramethyl trihydroxy hexadecane.

3. The water-in-oil emulsion composition according to claim 1 or 2, wherein the solid oil component is selected from the group consisting of solid fats hydrocarbons and waxes, lanolin fatty acid isopropyl, hexyl laurate, reduced lanolin, hard lanolin, POE lanolin alcohol ether, POE lanolin alcohol acetate, POE cholesterol ether, lanolin fatty acid polyethylene glycol, and POE hydrogenated lanolin alcohol ether, myristic acid, palmitic acid, stearic acid, and behenic acid, and cetyl alcohol, stearyl alcohol, behenyl alcohol, myristyl alcohol, and cetostearyl alcohols.

4. The water-in-oil emulsion composition according to any of claims 1 to 3, wherein the viscosity of the emulsion is 2.000 to 15.000 mPa·s.

## Patentansprüche

1. Wasser-in-Öl-Emulsions-Zusammensetzung, umfassend die unten beschriebenen Komponenten (A), (B) und (C), wobei sie die unten beschriebenen Bedingungen (1) bis (4) erfüllt;
Komponenten:
(A) 0,1 bis 5,0 Massen-% Glycerinisostearat und/oder Glycerinoleat in Bezug auf die Gesamtmenge der Zusammensetzung,
(B) eine wässrige Komponente, und
(C) eine Ölkomponente, enthaltend ein Isoparaffin mit 20 Kohlenstoffatomen oder weniger und eine feste Ölkomponente;
Bedingungen:
(1) der Anteil der internen wässrigen Phase, der erhalten wird, indem die Masse der wässrigen Komponente (B) durch die Summe der Masse der wässrigen Komponente (B) und der Masse der Ölkomponente (C) dividiert wird, beträgt 68 % oder mehr, und
(2) die Menge an Glycerinmonoisostearat und/oder Glycerinmonooleat, die in der Komponente (A) enthalten ist, beträgt 85 Massen-% oder mehr in Bezug auf die Gesamtmenge von (A),
(3) die in der Komponente (C) enthaltene Menge eines Isoparaffins mit 20 Kohlenstoffatomen oder weniger beträgt 30 Massen-% oder mehr in Bezug auf die Gesamtmenge des Kohlenwasserstofföls, und
(4) in der Wasser-in-Öl-Emulsion sind die Komponente (B) und die Komponente (A) in einem Mehrphasen-Gleichgewichtszustand vorhanden, wobei der Mehrphasen-Gleichgewichtszustand erhalten wird, indem die Komponente (B) und die Komponente (A) gemischt werden, und wobei bei dem Mehrphasen-Gleichgewichtszustand (i) bikontinuierliche kubische Flüssigkristalle, reverse hexagonale Flüssigkristalle und eine Wasserphase koexistent sind, oder (ii) bikontinuierliche kubische Flüssigkristalle, reverse hexagonale Flüssigkristalle, andere Phasen und eine Wasserphase koexistent sind.

2. Wasser-in-Öl-Emulsions-Zusammensetzung nach Anspruch 1, ferner umfassend eine Komponente (D):
Tetramethyltrihydroxyhexadecan.

3. Wasser-in-Öl-Emulsions-Zusammensetzung nach Anspruch 1 oder 2, wobei die feste Ölkomponente aus der Gruppe ausgewählt ist, die aus festen Fetten, Kohlenwasserstoffen und Wachsen, Lanolinfettsäureisopropyl, Hexyllaurat, reduziertem Lanolin, hartem Lanolin, POE-Lanolinalkoholether, POE-Lanolinalkoholacetat, POE-Cholesterinether, Lanolinfettsäurepolyethylenglycol und hydriertem POE-Lanolinalkoholether, Myristinsäure, Palmitinsäure, Stearinsäure, Behensäure und Cetylalkohol, Stearylalkohol, Behenylalkohol, Myristylalkohol und Cetostearylalkoholen besteht.

4. Wasser-in-Öl-Emulsions-Zusammensetzung nach einem der Ansprüche 1 bis 3, wobei die Viskosität der Emulsion 2.000 bis 15.000 mPa·s beträgt.

## Revendications

1. Composition d'émulsion eau dans l'huile comprenant les composants (A) (B) et (C) décrits ci-dessous, qui satisfait les conditions (1) à (4) décrites ci-dessous ;
Composants :
(A) 0,1 à 5,0 % en masse d'isostéarate de glycérol et/ou d'oléate de glycérol rapporté à la quantité totale de composition,
(B) un composant aqueux, et
(C) un composant huile contenant une isoparaffine ayant 20 atomes de carbone ou moins et un composant huile solide ;
Conditions :
(1) la proportion de phase aqueuse-interne, qui est obtenue en divisant la masse du composant aqueux (B) par la somme de la masse du composant aqueux (B) et de la masse du composant huile (C), est de 68 % ou plus, et
(2) la quantité de monoisostéarate de glycérol et/ou de monooléate de glycérol contenue dans le composant (A) est de 85 % en masse ou plus rapporté à la quantité totale de (A),
(3) la quantité d'isoparaffine ayant 20 atomes de carbone ou moins contenue dans le composant (C) est de 30 % en masse ou plus rapporté à la quantité totale d'huile hydrocarbonée, et
(4) dans l'émulsion eau dans huile, le composant (B) et le composant (A) sont présents dans un état d'équilibre multiphasique, dans lequel l'état d'équilibre multiphasique est obtenu en mélangeant le composant (B) et le composant (A), et dans lequel l'état d'équilibre multiphasique est (i) des cristaux liquides cubiques bicontinus, des cristaux liquides hexagonaux inverses, et une phase d'eau coexistent, ou (ii) des cristaux liquides cubiques bicontinus, des cristaux liquides hexagonaux inverses, d'autres phases, et une phase d'eau coexistent.

2. Composition d'émulsion eau dans l'huile selon la revendication 1, comprenant en outre un composant (D) : le tétraméthyl trihydroxy hexadécane.

3. Composition d'émulsion eau dans l'huile selon la revendication 1 ou 2, dans laquelle le composant huile solide est choisi dans le groupe consistant en les graisses hydrocarbures et cires solides, l'isopropyl acide gras de lanoline, le laurate d'hexyle, la lanoline réduite, la lanoline dure, l'éther d'alcool de lanoline POE, l'acétate d'alcool de lanoline POE, l'éther de cholestérol POE, le polyéthylène glycol d'acide gras de lanoline, et l'éther d'alcool de lanoline hydrogénée POE, l'acide myristique, l'acide palmitique, l'acide stéarique, et l'acide béhénique, et l'alcool cétylique, l'alcool stéarylique, l'alcool béhénylique, l'alcool myristique, et les alcools cétostéarylique.

4. Composition d'émulsion eau dans l'huile selon l'une quelconque des revendications 1 à 3, dans laquelle la viscosité d'émulsion est de 2 000 à 15 000 mPa.s.
